# EUROPEAN PATENT APPLICATION

(11) **EP 4 487 865 A1**
(43) Date of publication of application: **08.01.2025**
(21) Application number: 23763758.2
(22) Date of filing: 03.03.2023
(51) Int. Cl.: A61K 38/17, A61K 39/395, A61K 45/06, A61P 35/00

(54) **COMPOSITION FOR COMBI-THERAPY COMPRISING VEGF-GRAB AND PD-1 OR PD-L1 ANTAGONIST**

(30) Priority: 04.03.2022 KR 20220028068
(71) Applicant: Panolos Bioscience, Inc., Hwaseong-si Gyeonggi-do 18471 (KR)
(72) Inventor: LIM, Hyeseong, Seongnam-si Gyeonggi-do 13567 (KR); YANG, Hyun Gul, Asan-si Chungcheongnam-do 31579 (KR); YANG, Hannah, Ulsan 44242 (KR); KIM, Chan, Yongin-si Gyeonggi-do 16903 (KR); JEON, Hong Jae, Seongnam-si Gyeonggi-do 13528 (KR)
(74) Representative: Mewburn Ellis LLP
(86) International application number: PCT/KR2023/002973
(87) International publication number: WO 2023/167560

(57) **Abstract**

The present invention relates to a pharmaceutical composition for preventing or treating cancer, the composition containing VEGF-Grab as an active ingredient, and being intended for administration in combination with a PD-1 or PD-L1 antagonist.

## Description

### Technical Field

The present invention relates to a pharmaceutical composition for preventing or treating cancer, comprising VEGF-Grab as an active ingredient, for co-administration with a PD-1 or PD-L1 antagonist.

### Background Art

In order to proliferate and grow cancer cells, new blood vessels to supply them with oxygen and nutrients are needed, and vascular endothelial growth factor (VEGF) is known to play a pivotal role in the formation of new blood vessels. VEGF is a dimer of approximately 46 KDa composed of two subunits, and five types of VEGF have been identified in mammals (VEGF-A, VEGF-B, VEGF-C, VEGF-D, and PlGF). These VEGFs bind to three receptor tyrosine kinases (RTKs) known as VEGF receptor (VEGFR)-1, VEGFR-2, and VEGFR-3, and these VEGF receptors trigger cell migration, survival, and proliferation, and have the ability to transmit signals that form three-dimensional blood vessels which are not present in other RTKs, or regulate vascular permeability.

VEGF has been used as a target for cancer therapy since it was discovered that the expression of VEGF molecules is increased in tumor cells, and the receptor for VEGF is increased in tumor-infiltrating vascular endothelial cells, but both VEGF and its receptor are lowly expressed in normal cells which are not involved in angiogenesis.

As a result, new anticancer therapies have been developed that block the production of blood vessels that nourish cancer cells rather than the cancer cells themselves, and it has been reported that anti-VEGF receptor antibodies, soluble receptor constructs, antisense, RNA aptamers against VEGF, and low molecular weight VEGF receptor tyrosine kinase (RTK) inhibitors can be used to interfere with VEGF signaling. Indeed, anti-VEGF neutralizing antibodies have been shown to inhibit the growth of various human tumor cell lines in nude mice (Warren et al. J. Clin. Invest. 95: 1789-1797 (1995)). In addition, patent literatures relating to VEGF inhibitors have disclose a variety of VEGF inhibitors such as quinazoline derivatives as VEGF inhibitors (U.S. Patent No. 9040548), inhibitors of VEGF receptor and HGF receptor signaling for treating angiogenesis-mediated cytoproliferative diseases or inhibiting solid tumor growth (U.S. Patent No. 8470850), substances that inhibit angiogenesis by interfering with the binding of VEGF to its receptor, used in the treatment of diseases such as cancer (Korean Patent Publication No. 2003-0075947) .

On the other hand, the body's immune system regulates overall T lymphocyte function by regulating these co-stimulatory and co-inhibitory signals simultaneously with antigen recognition. This regulatory mechanism is called the immune checkpoint. The body's immune system detects tumor-specific neo-antigens, which are expressed due to changes such as mutations in tumor cells, and eliminates tumor cells or viral infectious agents.

However, some tumor cells, on the contrary, try to evade this immune attack by altering the tumor micro-environment to suppress immune function or to immune escape through T-cell immune tolerance or immuno-editing.

One such escape strategy is to inhibit the function of tumor-specific T-lymphocyte cells through alterations in immune checkpoint function, i.e., to activate inhibitory immune checkpoints on tumor cells to evade attack by tumor-specific T-lymphocyte cells. In this regard, monoclonal antibodies against PD-1 or ligand PD-L1 can be used to inhibit its function, thereby enhancing the activity and effectiveness of the inhibited tumor-specific T-lymphocyte cells to achieve antitumor effects.

Against this technical background, the inventors of the present invention, while researching cancer immunotherapy, identified that the combination of VEGF-Grab and PD-1 or PD-L1 antagonists delayed tumor growth and prevented cancer metastasis, and have completed the present invention.

The above information in this Related Art section is intended solely to enhance the understanding of the background of the present invention and may not include information of the prior art known to one having ordinary skill in the art.

### Summary of the Invention

It is an object of the present invention to provide a pharmaceutical composition for the prevention or treatment of cancer, comprising VEGF-Grab as an active ingredient, for co-administration with a PD-1 or PD-L1 antagonist.

Another object of the present invention is to provide a method of preventing or treating cancer administering a VEGF-Grab, or a pharmaceutical composition comprising the same as an active ingredient, in combination with a PD-1 or PD-L1 antagonist, or a pharmaceutical composition comprising the same.

Another object of the present invention is to provide a use for VEGF-Grab or a pharmaceutical composition comprising the same as an active ingredient, for preventing or treating cancer, in combination with a PD-1 or PD-L1 antagonist or a pharmaceutical composition comprising the same.

Another object of the present invention is to provide the use of VEGF-Grab or a pharmaceutical composition comprising the same as an active ingredient, in the manufacture of a medicament for preventing or treating cancer, in combination with a PD-1 or PD-L1 antagonist or a pharmaceutical composition comprising the same.

To achieve the above objectives, the present invention provides a pharmaceutical composition for the prevention or treatment of cancer, comprising as an active ingredient a fusion protein comprising vascular endothelial growth factor receptor 1 (VEGFR1) domain 2, VEGFR1 domain 3 and the Fc region of an antibody, for co-administration with a PD-1 or PD-L1 antagonist.

The present invention also provides a method of preventing or treating cancer, administering VEGF-Grab or a pharmaceutical composition comprising the same as an active ingredient, in combination with a PD-1 or PD-L1 antagonist or a pharmaceutical composition comprising the same.

The present invention also provides a use of VEGF-Grab or a pharmaceutical composition comprising the same as an active ingredient thereof, for preventing or treating cancer, in combination with a PD-1 or PD-L1 antagonist or a pharmaceutical composition comprising the same.

The present invention also provides the use of a pharmaceutical composition comprising VEGF-Grab or a pharmaceutical composition comprising the same as an active ingredient, in the manufacture of a medicament for preventing or treating cancer, in combination with a PD-1 or PD-L1 antagonist or a pharmaceutical composition comprising the same.

### Brief Description of the Drawings

FIG. 1 provides an overview of the combination immunotherapy of PB101 and PD-L1 immune checkpoint inhibitor in the MC38 colon cancer model.
FIG. 2 illustrates the tumor growth retardation effect in the PB101 and αPD-L1 combination group.
FIG. 3 provides an overview of the combination immunotherapy of PB101 and PD-L1 immune checkpoint inhibitor in the Hepa-VEGF liver cancer model.
FIG. 4 illustrates the tumor growth retardation effect in the PB101 and αPD-L1 combination group.
FIG. 5 illustrates the survival benefit in the PB101 and αPD-L1 combination group.
FIG. 6 shows an overview of the rechallenge experiment of cancer cells after combination immunotherapy of PB101 and PD-L1 immune checkpoint inhibitor in the Hepa-VEGF liver cancer model.
FIG. 7 shows the results of confirming that when cancer is rechallenged in the combination group, it does not grow.
FIG. 8 shows the results of confirming that cancer does not metastasize when rechallenged in the combination group.

### Detailed Description of the Invention and Preferred Embodiments of the Invention

Unless otherwise defined, all technical and scientific terms used herein shall have the same meaning as commonly understood by those skilled in the art. In general, the nomenclature used herein is well known and in common use in the art.

In one aspect, the present invention relates to a pharmaceutical composition for the prevention or treatment of cancer, comprising as an active ingredient a fusion protein comprising vascular endothelial growth factor receptor 1 (VEGFR1) domain 2, VEGFR1 domain 3 and the Fc region of an antibody, for co-administration with a PD-1 or PD-L1 antagonist.

In another aspect, the present invention relates to a method of preventing or treating administering a fusion protein comprising VEGFR1 (vascular endothelial growth factor receptor 1) domain 2, VEGFR1 domain 3 and the Fc region of an antibody or a pharmaceutical composition comprising the same, in combination with a PD-1 or PD-L1 antagonist or a pharmaceutical composition comprising the same.

In another aspect, the present invention relates to the use of a fusion protein comprising vascular endothelial growth factor receptor 1 (VEGFR1) domain 2, VEGFR1 domain 3 and the Fc region of an antibody, or a pharmaceutical composition comprising the same as an active ingredient, for preventing or treating cancer in combination with a PD-1 or PD-L1 antagonist or a pharmaceutical composition comprising the same.

In another aspect, the present invention relates to the use of a fusion protein comprising vascular endothelial growth factor receptor 1 (VEGFR1) domain 2, VEGFR1 domain 3 and the Fc region of an antibody, or a pharmaceutical composition comprising the same as an active ingredient, in the manufacture of a medicament for preventing or treating cancer, in combination with a PD-1 or PD-L1 antagonist or a pharmaceutical composition comprising the same.

Hereinafter, the present invention is described in more detail.

### VEGF-Grab

The present invention comprises a fusion protein comprising vascular endothelial growth factor receptor 1 (VEGFR1) domain 2, VEGFR1 domain 3, and the Fc region of an antibody.

"VEGFR1" (vascular endothelial growth factor receptor 1) is the receptor for vascular endothelial growth factor (VGEF), which can stimulate cell division, migration, and differentiation by activating the receptor's tyrosine kinase. In addition, VEGFR1 domain 2 and domain 3 are domains that recognize VEGF.

VEGFR1 domains 2 and 3 are not limited to their origin or sequence and may include wild-type or active variants thereof, as they may be different in the amino acid sequence of proteins that exhibit activity in accordance with species. VEGFR1 domain 2 of the present invention may comprise the amino acid sequence of SEQ ID NO: 1, and VEGFR1 domain 3 may comprise the amino acid sequence of SEQ ID NO: 2.

The amino acid sequence of VEGFR1 domain 2 (SEQ ID NO: 1):

EFGRPFVEMYSEIPEIIHMTEGRELVIPCRVTSPNITVTLKKFPLDTLIPDGKRII

WDSRKGFIISNATYKEIGLLTCEATVNGH

The amino acid sequence of VEGFR1 domain 3 (SEQ ID NO: 2):

LYKTNYLTHRQTNTIIDVQISTPSPVTLLRGHTLVLNCTATTPLNTRVQMTWSYPD
EKNKNASVRRRIDQSNSHANIFYSVLTIDKMQNKDKGLYTCRVRSGPSFKSVNTSVHIYDKA

"Antibody Fc region" means the heavy chain invariant region 2 (CH2) and heavy chain invariant region 3 (CH3), excluding the heavy chain and light chain variable regions, the heavy chain invariant region 1, (CH1) and light chain invariant region 1 (CL1) of an immunoglobulin, and may also include a hinge region in the heavy chain invariant region. The antibody Fc region is a biodegradable polypeptide that is metabolized in vivo and is therefore safe for use as a drug carrier. In addition, the immunoglobulin Fc region has a relatively small molecular weight compared to the total molecule of the immunoglobulin, which is advantageous in terms of preparation, purification and yield of the conjugate, and since the amino acid sequence varies from antibody to antibody, the homogeneity of the material is greatly increased by eliminating the Fab portion, which exhibits high inhomogeneity, and the possibility of inducing blood antigenicity is reduced.

The Fc region of the antibody may be, but is not limited to, an Fc region derived from IgA, IgD, IgE, IgG, IgM, or made by a combination thereof or a hybrid thereof, or more specifically derived from IgG or IgM, which is the most abundant in human blood, and more specifically human-derived IgG1.

The fusion protein may refer to a protein comprising VEGFR1 domain 2 (R1D2) - VEGFR1 domain 3 (R1D3) - hinge - Fc region (CH2 and CH3) of a human antibody, and may be used interchangeably with the term "VEGF-Grab".

The fusion protein binds to VEGF, the ligand of VEGFR1 (vascular endothelial growth factor receptor 1), and inhibits the activity by binding to VEGF-A, VEGF-B or PlGF. The fusion protein may comprise an amino acid sequence of SEQ ID NO: 3.

Amino acid sequence of the VEGF-Grab fusion protein (SEQ ID NO: 3):

EFGRPFVEMYSEIPEIIHMTEGRELVIPCRVTSPNITVTLKKFPLDTLIPDGKRII

WDSRKGFIISNATYKEIGLLTCEATVNGHLYKTNYLTHRQTNTIIDVQISTPSPVTLLRGHT

LVLNCTATTPLNTRVQMTWSYPDEKNKNASVRRRIDQSNSHANIFYSVLTIDKMQNKDKGLY

TCRVRSGPSFKSVNTSVHIYDKALEDKTHTCPPCPAPELLGGPSVFLFPPKPKDTLMISRTP

EVTCVVVDVSHEDPEVKFNWYVDGVEVHNAKTKPREEQYNSTYRVVSVLTVLHQDWLNGKEY

KCKVSNKALPAPIEKTISKAKGQPREPQVYTLPPSREEMTKNQVSLTCLVKGFYPSDIAVEW

ESNGQPENNYKTTPPVLDSDGSFFLYSKLTVDKSRWQQGNVFSCSVMHEALHNHYTQKSLSL

SPGK

In one example of the present invention, the VEGF-Grab fusion protein is named "PB101".

The fusion protein may be linked from the N terminus in the order of VEGFR1 domain 2, VEGFR1 domain 3 and the antibody fragment, or in the order of VEGFR1 domain 3, VEGFR1 domain 2 and the antibody fragment, wherein in the fusion protein, VEGFR1 domain 2, VEGFR1 domain 3 and the Fc region of the antibody may be linked to each other directly or via a linker.

The linker is not particularly limited insofar as it enables the fusion protein to exhibit activity, but in particular, it can use amino acids such as glycine, alanine, leucine, isoleucine, proline, serine, threonine, asparagine, aspartic acid, cysteine, glutamine, glutamic acid, lysine, or arginine. More specifically, amino acids such as valine, leucine, aspartic acid, glycine, alanine, proline, and the like can be used and linked in groups of multiple numbers, and more specifically, amino acids such as glycine, valine, leucine, aspartic acid, and the like can be used and linked in groups of one to twenty, taking into account the ease of genetic manipulation.

Specifically, the linker may be a peptide linker, and may comprise about 5-25 amino acid (aa) residues, or more specifically, may comprise about 5-30 aa residues. For example, it may include, but is not limited to, hydrophilic amino acids such as glycine and/or serine.

The linker may comprise, for example, a glycine linker (G,Gly)ₚ (where p is 1 to 10), a GS linker (GₙS)ₘ (where n and m are 1 to 10, respectively) to provide structural flexibility. Specifically, the linker may comprise GGGGS or (GGGGS)₂, or may comprise 1-10 aa of glycine in (G,Gly)ₚ where p is 1 to 10.

### PD-1 or PD-L1 antagonist

The PD-1 antagonist includes an agent capable of inhibiting the binding of PD-L1 to PD-1. For example, the PD-1 antagonist may be an anti-PD-1 antibody.

The PD-L1 antagonist includes an agent capable of inhibiting the binding of PD-L1 to PD-1. For example, the PD-L1 antagonist may be an anti-PD-Ll antibody.

"Antibody" means an anti-PD-1 antibody or an anti-PD-Ll antibody that specifically binds to PD-1 or PD-L1. The scope of the present invention includes antigen-binding fragments of the antibody molecules, as well as complete antibody forms that specifically bind to PD-1 or PD-L1.

A complete antibody has a structure with two full-length light chains and two full-length heavy chains, each of which is linked to the heavy chain by a disulfide bond.

As used herein, the term "heavy chain" refers to both full-length heavy chains comprising a variable region domain VH comprising an amino acid sequence having sufficient variable region sequence to confer specificity to an antigen and three invariant domains CH1, CH2, and CH3, and fragments thereof. Also, as used herein, the term "light chain" refers to both full-length light chains comprising a variable region domain VL comprising an amino acid sequence having sufficient variable region sequence to confer specificity to an antigen and an invariant region domain CL, and fragments thereof.

The total antibody comprises subtypes of IgA, IgD, IgE, IgM and IgG, and in particular IgG comprises IgG1, IgG2, IgG3 and IgG4. The heavy chain invariant region has the gamma (γ), mu (µ), alpha (α), delta (δ), and epsilon (ε) types with subclasses gamma1 (γ1), gamma2 (γ2), gamma3 (γ3), gamma4 (γ4), alpha1 (α1), and alpha2 (α2). Invariant regions of light chains have kappa (κ) and lambda (λ) types.

Antigen-binding fragment or antibody fragment of an antibody refers to a fragment that possesses antigen-binding function, including Fab, F(ab'), F(ab')₂, and Fv. Among the antibody fragments, Fab has a structure with variable regions of light and heavy chains, an invariant region of light chain and the first invariant region (CH1) of heavy chain, and has one antigen-binding site. Fab' differs from Fab in that it has a hinge-region containing one or more cysteine residues at the C-terminus of the heavy chain CH1 domain. F(ab')₂ is generated by disulfide bonding of cysteine residues in the hinge region of Fab'.

Fv corresponds to the smallest antibody fragment that has only a heavy chain variable region and a light chain variable region. Two-chain Fv has a non-covalently linked heavy and light chain variable regions, while single-chain Fv (scFv) has a covalently linked heavy and light chain variable regions, usually via a peptide linker, or directly at the C-terminus, which can form a dimer-like structure like double-chain Fv. These antibody fragments can be produced by using proteinases (e.g., restriction cleavage of the intact form of the antibody with papain yields Fab and cleavage with pepsin yields F(ab')₂) or by genetic recombination techniques.

"Fv" fragments are antibody fragments that contain complete antibody recognition and binding sites. These regions are dimers of one heavy chain variable domain and one light chain variable domain.

A "Fab" fragment includes variable and invariant domains in the light chain, and variable and first invariant domains (CH1) in the heavy chain. F(ab')₂ antibody fragments typically include a pair of Fab' fragments covalently linked by cysteines in a hinge region present at the C-terminus of the Fab' fragment.

A "single-chain Fv (scFv)" antibody fragment has a structure consisting of a single polypeptide chain containing the VH and VL domains of an antibody. A polypeptide linker may be additionally comprised between the VH and VL domains to allow the scFv to form the desired structure for binding to the antigen.

In the present invention, the antibodies include, but are not limited to, monoclonal antibodies, multispecific antibodies, human antibodies, humanized antibodies, chimeric antibodies, scFvs, Fab fragments, F(ab')₂ fragments, disulfide-bound Fvs (sdFvs) and anti-idiotype (anti-Id) antibodies, or epitope-bound fragments of the above antibodies.

The heavy chain invariant region may be selected from any one of the following isotypes: gamma (γ), mu (µ), alpha (α), delta (δ), or epsilon (ε). For example, the invariant region is gamma 1 (IgG1), gamma 2 (IgG2), gamma 3 (IgG3), or gamma 4 (IgG4). The light chain invariant region may be of kappa or lambda type.

The monoclonal antibodies refer to antibodies obtained from a substantially homogeneous population of antibodies, in other words, indicate the same except for possible naturally occurring mutations in which the individual antibodies in the population may be present in trace amounts. Monoclonal antibodies are highly specific, so they are directed against a single antigenic site. In contrast to conventional (polyclonal) antibodies, which typically contain different antibodies directed against different determinants (epitopes), each monoclonal antibody is directed against a single determinant on the antigen.

An "epitope" is a protein determinant to which an antibody can specifically bind. Epitopes typically consist of a group of chemically active surface molecules, such as amino acids or sugar side chains, and typically have specific charge characteristics as well as specific three-dimensional structural features. Steric and amorphous epitopes are distinguished in that binding to the former is lost in the presence of a denaturing solvent, but not to the latter.

Non-human (e.g., murine) antibodies in their "humanized" form are chimeric antibodies containing a minimal sequence derived from a non-human immunoglobulin. In most cases, a humanized antibody is a human immunoglobulin (recipient antibody) in which residues from the hypervariable region of the recipient is replaced with residues from the hypervariable region of a non-human species (donor antibody), such as mouse, rat, rabbit, or non-human primate, that possesses the desired specificity, affinity, and ability.

The "human antibody" means a molecule derived from a human immunoglobulin, wherein the entire amino acid sequence constituting the antibody including complementary crystal regions and structural regions is composed of human immunoglobulin.

"Chimeric" antibodies (immunoglobulins) in which a portion of the heavy and/or light chain is identical or homologous to a corresponding sequence in an antibody derived from a particular species or belonging to a particular antibody class or subclass, while the remaining chain(s) are identical or homologous to a corresponding sequence in an antibody derived from another species or belonging to another antibody class or subclass, and fragments of the antibodies exhibiting the intended biological activity are comprised.

As used herein, "variable region" of an antibody refers to the light chain and heavy chain portions of the antibody molecule comprising the amino acid sequences of the complementarity determining region (CDR; i.e., CDR1, CDR2, and CDR3) and the framework region (FR). VH refers to the variable domain of the heavy chain. VL refers to the variable domain of the light chain.

"Complement determining region (CDR)" refers to the amino acid residues in an antibody variable domain that are required for antigen binding. Each variable domain typically has three CDR regions, identified as CDR1, CDR2, and CDR3.

"Framework regions (FRs)" are variable domain residues other than CDR residues. Each variable domain typically has four FRs, identified as FR1, FR2, FR3, and FR4.

In one example of the present invention, the anti-PD-1 antibody or anti-PD-Ll antibody may be, but is not limited to, 10F.9G2, specifically, a mouse PD-L1 antibody.

The anti-PD-1 antibody may be, for example, AMP-224, GLS-010, PDR-001, Pidilizumab, PF-06801591, Genolimzumab, CA-170, MEDI-0680, JS-001, LZM-009, AK-103, Nivolumab, Dostarlimab, Pembrolizumab, Sintilimab, Tislelizumab, Toripalimab, Camrelizumab, or Cemiplimab.

The anti-PD-Ll antibody may be, for example, CX-072, WBP-3155, KN035, A167, Cosibelimab, Atezolizumab, Avelumab, Durvalumab, Adebrelimab, or BMS-936559.

### Nucleic Acid Molecule, Expression Vector, and Host Cell

Provided is an isolated nucleic acid molecule encoding the fusion protein or the antagonist.

The term "nucleic acid" encompasses DNA (gDNA and cDNA) and RNA molecules, and the nucleotide, the basic constitutional unit of a nucleic acid, includes not only naturally occurring nucleotides, but also analogues with sugar or base site modifications. The sequences of the nucleic acids encoding the heavy and light chain variable regions of the present invention may be modified. The modifications include additions, deletions, or non-conservative or conservative substitutions of nucleotides.

An expression vector comprising the isolated nucleic acid molecule is provided. As used herein, the term "vector" refers to a means for expressing a target gene in a host cell, including viral vectors such as plasmid vectors, cosmid vectors, bacteriophage vectors, adenoviral vectors, retroviral vectors, adeno-associated viral vectors, and the like. Components of a vector typically include, but are not limited to, one or more of the following: a signal sequence, a replication site, one or more antibiotic resistance marker genes, an enhancer element, a promoter, and a transcription termination sequence. The nucleic acid encoding the antibody is operatively linked to the promoter and transcription termination sequence.

"Operatively linked" means a functional association between a nucleic acid expression regulatory sequence (e.g., a promoter, signal sequence, or array of transcriptional regulator binding sites) and another nucleic acid sequence, whereby the regulatory sequence regulates the transcription and/or decoding of the other nucleic acid sequence.

In the case of prokaryotic cells as hosts, it is common to include a strong promoter capable of driving transcription (e.g., a tac promoter, lac promoter, lacUV5 promoter, lpp promoter, pLλ, promoter, pRλ promoter, rac5 promoter, amp promoter, recA promoter, SP6 promoter, trp promoter, and T7 promoter), a rhizosome binding site for initiation of decoding, and a transcription/decoding termination sequence. In addition, for example, in the case of eukaryotic cells as hosts, a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, vaccinia virus 7.5K promoter, SV40 promoter, cytomegalovirus (CMV) promoter, tk promoter of HSV, mouse mammary tumor virus (MMTV) promoter, LTR promoter of HIV, Moloney virus promoter, Epstein-Barr virus (EBV) promoter, and Roos Sakoma virus (RSV) promoter) may be utilized and they typically have a polyadenylation sequence as a transcription termination sequence.

As used herein, the term "expression of a heterologous nucleic acid sequence" or "expression" of a target protein refers to the transcription of the inserted DNA sequence, the translation of the mRNA transcript, and the production of a fusion protein product or antibody or antibody fragment.

The vector comprises as selective markers antibiotic resistance genes conventionally used in the art, for example, resistance genes for ampicillin, gentamicin, carbenicillin, chloramphenicol, streptomycin, kanamycin, geneticin, neomycin, and tetracycline.

A host cell comprising the expression vector is provided.

As used herein, the term "host cell" refers to prokaryotic and eukaryotic cells into which recombinant expression vectors may be introduced. As used herein, the terms "transformed" and "transfected" refer to the introduction of a nucleic acid (e.g., vector) into a cell by any of the many techniques known in the art.

Prokaryotic host cells such as strains of the Bacillus genus, such as *Escherichia coli, Bacillus subtilus,* and *Bacillus thuringiensis;* Streptomyces; Pseudomonas (e.g., *Pseudomonas putida); Proteus mirabilis;* or Staphylococcus (e.g., *Staphylococcus carnosus)* can be used.

However, animal cells are of most interest, and examples of useful host cell lines may include, but not limited to, COS-7, BHK, CHO, CHOK1, DXB-11, DG-44, CHO/-DHFR, CV1, COS-7, HEK293, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S and HT1080.

The host cells can be cultured in a variety of media. Any of the commercially available media can be used as culture media without limitation. All other necessary supplements known to those skilled in the art may also be included in suitable concentrations. Culture conditions, e.g., temperature, pH, etc., are already used with host cells selected for expression and it is apparent to those skilled in the art.

Recovery of the fusion protein or the antagonist can be accomplished by removing impurities, for example by centrifugation or ultrafiltration, and purifying the resulting product, for example by affinity chromatography. Additional other purification techniques may be used, for example, anion or cation exchange chromatography, hydrophobic interaction chromatography, hydroxyapatite chromatography, and the like.

### Pharmaceutical composition

The pharmaceutical composition according to the present invention can be administered by any route. The composition of the invention can be provided to an animal by any suitable means, either directly (e.g., by injection, implantation or local administration to a tissue site, or by topical administration) or systemically (e.g., parenterally or orally). When the composition of the present invention is administered parenterally, such as intravenously, subcutaneously, ophthalmically, intraperitoneally, intramuscularly, orally, rectally, intraorbitally, intracerebrally, intracranially, intraspinally, intraventricularly, intrathecally, intracistenally, or intracapsularly, intranasally, or via aerosol administration, the pharmaceutical composition may comprise, for example, a portion of an aqueous or physiologically applicable body fluid suspension or solution. Accordingly, a carrier or vehicle is physiologically acceptable and can be added to the composition and delivered to the patient. Thus, a carrier such as a body fluid for a formulation may typically include normal saline.

Furthermore, the frequency of administration is depend on the pharmacokinetic parameters of the VEGF-Grab, PD-1 or PD-L1 antagonist in the formulation used. Typically, the clinician will administer the pharmaceutical composition until a dose is reached that achieves the intended effect. Accordingly, the pharmaceutical composition may be administered as a single dose, as two or more time-spaced doses, or as a continuous infusion via an implanted device or catheter. Further refinement of the appropriate dosage is routinely made by those skilled in the art and falls within the scope of work routinely performed by them.

Furthermore, the unit dose is 0.01 µg/kg to 100 mg/kg in humans, more specifically 1 µg/kg to 10 mg/kg. Although the above amounts are optimal, they may vary depending on the disease to be treated and the presence or absence of side effects, and the optimal dosage may be determined using conventional experiments. Administration of the fusion protein may be by periodic bolus injections or by continuous intravenous, subcutaneous, or intraperitoneal administration from an external reservoir (e.g., intravenous bag) or internal reservoir (e.g., bioerodable implant). The PD-1 or PD-L1 antagonist may be administered intravenously or subcutaneously.

The dosage of the fusion protein may be administered at 1 mg/kg to 15 mg/kg. Specifically, it can be administered at 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, or 15 mg/kg.

The dosage of the PD-1 or PD-L1 antagonist may be administered at 1 mg/kg to 20 mg/kg. Specifically, the PD-1 or PD-L1 antagonist may be administered at a dose of 1 mg/kg, 2 mg/kg, 3 mg/kg, 4 mg/kg, 5 mg/kg, 6 mg/kg, 7 mg/kg, 8 mg/kg, 9 mg/kg, 10 mg/kg, 11 mg/kg, 12 mg/kg, 13 mg/kg, 14 mg/kg, 15 mg/kg, 16 mg/kg, 17 mg/kg, 18 mg/kg, 19 mg/kg, 20 mg/kg.

### Combination

Preferably, VEGF-Grab, PD-1 or PD-L1 antagonists have complementary activities, so that they do not adversely affect each other.

The composition according to the present invention can be (1) co-formulated into a composite formulation and administered or delivered simultaneously; or (2) administered or delivered as a separate formulation, either simultaneously or in sequence.

For the combined formulation, the VEGF-Grab, PD-1 or PD-L1 antagonist may be present in the same composition. The formulation may be, for example, but not limited to, a dried powder composition, solution, or suspension.

VEGF-Grab, PD-1, or PD-L1 antagonists may be administered simultaneously or sequentially. VEGF-Grab, PD-1, or PD-L1 antagonists are typically administered separately from each other and may be administered simultaneously or sequentially. If administered sequentially, they may be administered in two or more doses. When administered sequentially, one of the VEGF-Grab, PD-1, or PD-L1 antagonists may be followed by the other, or alternating doses of the VEGF-Grab, PD-1, or PD-L1 antagonist may be administered with an interval between the two doses of the VEGF-Grab, PD-1, or PD-L1 antagonist.

Suitable dosages for the above agents may be those currently in use in the art, and may be reduced due to combined use.

### Administration of the composition

The composition of the present invention can be administered by any route suitable for the condition to be treated. Suitable routes include oral, parenteral (including subcutaneous, intramuscular, intravenous, intra-arterial, inhalation, intradermal, intrathecal, intrathecal, and infusion techniques), transdermal, rectal, intranasal, topical (including cheek and sublingual), vaginal, intraperitoneal, intrapulmonary, and intranasal administration. Topical administration may include utilizing transdermal administration, such as transdermal patches or iontophoresis devices. The formulation of the drug is discussed in the following literature [Reference: Remington's Pharmaceutical Sciences, 18th Ed., (1995) Mack Publishing Co., Easton, PA]. Other examples of drug formulations are described in the following literature [Reference: Liberman, H. A. and Lachman, L., Eds., Pharmaceutical Dosage Forms, Marcel Decker, Vol 3, 2nd Ed., New York, NY] .

The preferred route may vary, for example, depending on the condition of the recipient. If administered orally, it may be formulated as a pill, capsule, tablet, or the like, with a pharmaceutically acceptable carrier, excipient, or excipient. For parenteral administration, it can be formulated as a unit dose injectable, with a pharmaceutically acceptable parenteral vehicle or diluent.

### Prevention or treatment

The composition of the present invention is used in the prevention or treatment of cancer, and more specifically in cancer immunotherapy.

The cancers include both solid and blood cancers, and more specifically include, but are not limited to, liver cancer, colorectal cancer, lung cancer, pancreatic cancer, non-small cell lung cancer, colon cancer, bone cancer, skin cancer, head or neck cancer, melanoma of the skin or eye, uterine cancer, ovarian cancer, rectal cancer, stomach cancer, anorectal cancer, breast cancer, fallopian tube carcinoma, endometrial carcinoma, cervical carcinoma, vaginal carcinoma, carcinoma of the vulva, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or urinary tract cancer, renal cell carcinoma, renal pelvic carcinoma, central nervous system (CNS) tumor, primary central nervous system lymphoma, spinal cord tumor, brain stem glioma, pituitary adenoma, intrahepatic biliary tract cancer, medulloblastoma, and pancreatic neuroendocrine tumor, and more specifically, solid tumors among the above cancers.

The cancer may be, for example, but not limited to, colorectal cancer or liver cancer.

The present invention provides the use for the prevention or treatment of cancer, and more specifically for cancer immunotherapy.

The present invention provides a method for the prevention or treatment of cancer, and more particularly, a method for immunotherapy of cancer. The subject may be an individual suffering from cancer. Further, the individual may be a mammal, preferably a human.

"Treatment" refers to any sign of success in any subjective or objective parameter, such as mitigation; remission; production of symptoms or impairments that are more acceptable to the patient, and reduction in the pathology or condition; slowing of the rate of regression or decline; production of a final point of regression that is less debilitating; or treatment or amelioration of an impairment, pathology, or condition, including an improvement in the patient's physical or mental well-being. Treatment or improvement of symptoms may be based on objective or subjective parameters, including physical examination, neuropsychiatric examination, and/or psychiatric evaluation.

An "effective amount" is generally an amount sufficient to reduce the severity or frequency of a symptom, eliminate a symptom or its underlying cause, prevent the occurrence of a symptom or its underlying cause, or ameliorate or correct an impairment resulting from or related to a disease state. In some examples, an effective amount is a therapeutically effective amount or a prophylactically effective amount. A "therapeutically effective amount" is an amount sufficient to correct a disease state or symptom, particularly a condition or symptom associated with a disease state, or to prevent, hinder, delay, or reverse the progression of a disease state or any other undesirable symptom associated in any way with a disease state. A "prophylactic effective amount" is an amount of a pharmaceutical composition that, when administered to a subject, has the intended prophylactic effect, e.g., prevention or delay of the onset of a disease state, or reduction in the likelihood of the onset (or recurrence) of a disease state or associated symptoms.

### Product

Kits comprising a VEGF-Grab, and PD-1 or PD-L1 antagonist useful in the treatment of any of the above-mentioned cancers may be provided. Such kits may include a single container when co-formulated into a composite formulation. Such kits include a container comprising each of a VEGF-Grab, or PD-1 or PD-L1 antagonist.

The kit may be associated with the container, or further comprise a cover or package insert thereon. The term "package insert" may refer to the instructions customarily included within the commercial package of a therapeutic product, and includes information regarding indications, utilization, dosage, administration, contraindications and/or warnings regarding use of such therapeutic product. Suitable containers include, for example, bottles, vials, syringes, blister packs, and the like. The container can be made of various materials, for example glass or plastic. The labeling or package insert indicates that the composition is used for the prevention or treatment of a selected disease, for example cancer, or for cancer immunotherapy. The composition may further comprise a second container comprising a pharmaceutically acceptable buffer, for example, bacteriostatic water for injection (BWFI), phosphate buffered saline, Ringer's solution or dextrose solution. Other materials which are desirable from a commercial and user perspective may be further included, such as other buffers, diluents, fillers, needles and syringes.

The method may further include instructions for administering the VEGF-Grab, or PD-1 or PD-L1 antagonist simultaneously, sequentially, or separately to a patient in need thereof.

Hereinafter, the present invention will be described in detail with reference to examples to facilitate understanding. However, examples according to the present invention may be modified in many other ways, and the scope of the present invention should not be construed as being limited to the following examples.

### Example 1: Combination immunotherapy of PB101 and PD-L1 immune checkpoint inhibitor in MC38 colon cancer model

### 1.1 Method

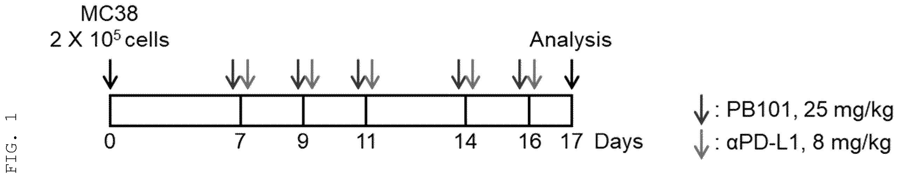

Eight-week-old C57BL/6 immunopreserved mice were injected subcutaneously with 2 × 10⁵ MC38 colon cancer cell lines in the right flank. When the tumor size reached approximately 50 mm3, drug administration proceeded via i.p. (intraperitoneal injection) (FIG. 1).

Tumor size was measured in all groups, and euthanasia was performed using CO₂ gas at the end of observation.

### 1.2 Results

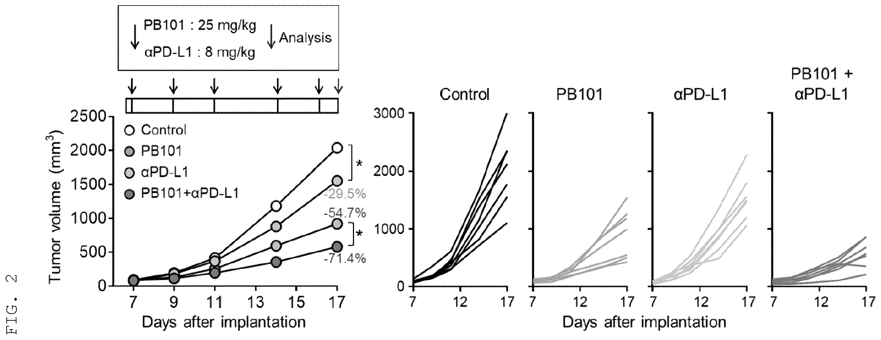

In the MC38 colon cancer model, it was observed that the αPD-L1 (10F.9G2, a mouse PD-L1 antibody) group showed 29.5% of a tumor growth suppression, and PB101 group showed 54.7%, as compared to the control group, and in particular, a significant increase in tumor growth suppression to 71.4% was shown in the PB101 and αPD-L1 combination group (FIG. 2).

Combination treatment of PB101 and αPD-L1 in the MC38 colon cancer model increased the anti-tumor response effect compared to the single treatment group.

### Example 2: Combination immunotherapy of PB101 and PD-L1 immune checkpoint inhibitor in a Hepa-VEGF liver cancer model

### 2.1 Method

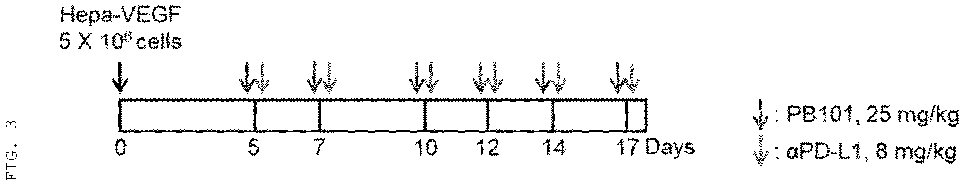

Eight-week-old C57BL/6 immunopreserved mice were injected subcutaneously with 5 × 10⁶ Hepa-VEGF liver cancer cell lines in the right flank. When the tumor size reached approximately 50 mm³, drug administration proceeded via i.p. (intraperitoneal injection) (FIG. 3).

Tumor size was measured in all groups, and survival was followed up to 85 days to ensure continued effects.

### 2.2 Results

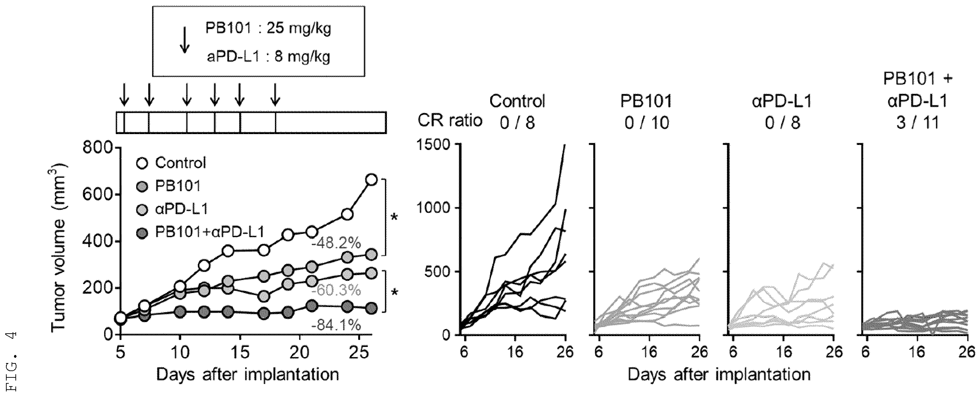

In the Hepa-VEGF liver cancer model, it was observed that the αPD-L1 group showed 48.2% of a tumor growth suppression, and the PB101 group showed 60.3% of a tumor growth suppression, as compared to the control group, and in particular, a significant increase in tumor growth suppression to 84.1% was shown in the PB101 and αPD-L1 combination group. In addition, complete tumor regression was observed in 27.2% (3/11) of the PB101 and αPD-L1 combination group, unlike the other experimental groups (FIG. 4).

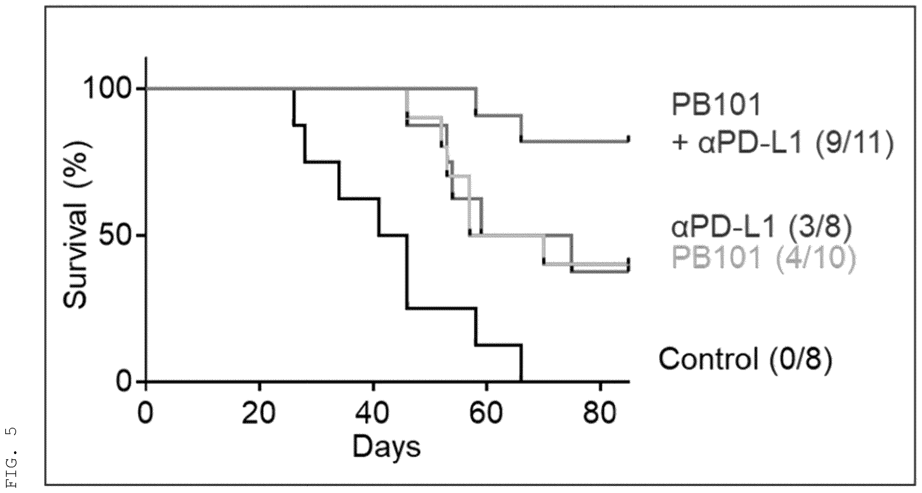

After the first two weeks of drug administration, a survival benefit was observed for both single administration and co-administration after drug withdrawal and monitoring for 60 days (FIG. 5).

### Example 3: Cancer cell rechallenge experiment after combination immunotherapy with PB101 and PD-L1 immune checkpoint inhibitor in a Hepa-VEGF liver cancer model

### 3.1 Method

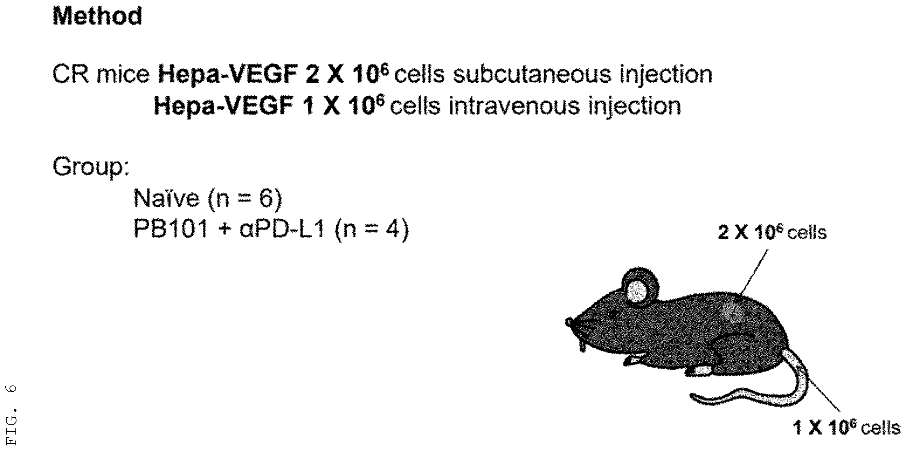

After 2 experiments, 4 mice with CR and near CR in the PB101 + aPD-L1 combination group were selected and injected subcutaneously with 2 × 10⁶ Hepa-VEGF liver cancer cell lines and simultaneously injected intravenously with 1 × 10⁶ Hepa-VEGF liver cancer cell lines. Six mice in the combination group were injected with cancer cells by the same method, followed up for 20 days, and euthanized (FIG. 6).

### 3.2 Results

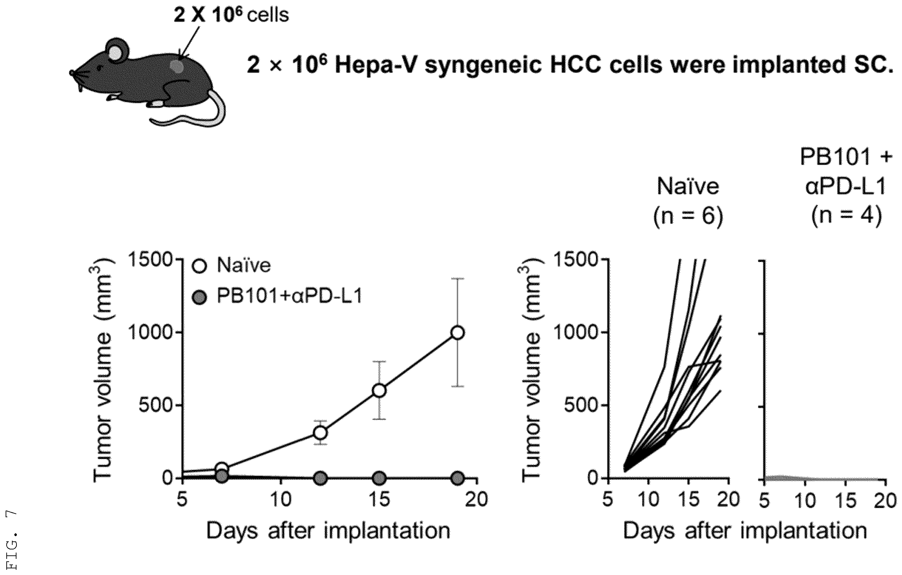

When cancer was rechallenged in the combination group, unlike the control group, the cancer did not grow (FIG. 7), which is presumed to be due to the formation of immune memory.

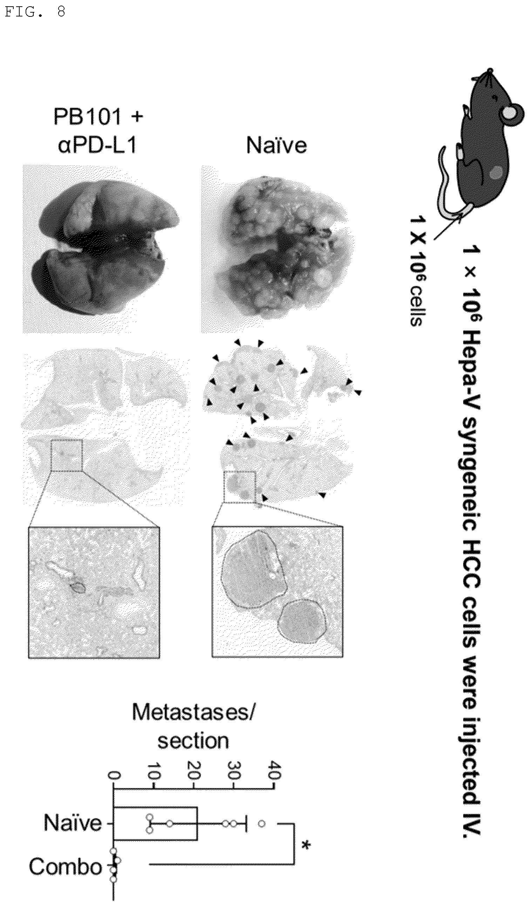

In addition, for intravenously injected cancer cells, metastasis to the lungs was identified in the control group but was rarely seen in the combination group (FIG. 8). This is likely due to induce long-lasting antitumor immune memory against recurrence and metastasis.

### Industrial Applicability

Pharmaceutical composition according to the present invention can be used to delay tumor growth and prevent metastasis of cancer.

While the foregoing has described in detail certain aspects of the present invention, it will be apparent to one of ordinary skill in the art that these specific descriptions are merely preferred examples and are not intended to limit the scope of the invention. Accordingly, the substantial scope of the invention will be defined by the appended claims and their equivalents.

**Sequence listing free text**

Electronic file attached.

## Claims

1. A pharmaceutical composition for the prevention or treatment of cancer, comprising as an active ingredient a fusion protein comprising VEGFR1 (vascular endothelial growth factor receptor 1) domain 2, VEGFR1 domain 3, and the Fc region of an antibody, for co-administration with a PD-1 antagonist or PD-L1 antagonist.

2. The pharmaceutical composition according to claim 1, wherein the VEGFR1 domain 2 comprises an amino acid sequence of SEQ ID NO: 1.

3. The pharmaceutical composition according to claim 1, wherein the VEGFR1 domain 3 comprises an amino acid sequence of SEQ ID NO: 2.

4. The pharmaceutical composition according to claim 1, wherein the PD-1 or PD-L1 antagonist is an anti-PD-1 antibody or an anti-PD-Ll antibody.

5. The pharmaceutical composition according to claim 4, wherein the anti-PD-1 antibody is AMP-224, GLS-010, PDR-001, Pidilizumab, PF-06801591, Genolimzumab, CA-170, MEDI-0680, JS-001, LZM-009, AK-103, Nivolumab, Dostarlimab, Pembrolizumab, Sintilimab, Tislelizumab, Toripalimab, Camrelizumab, or Cemiplimab.

6. The pharmaceutical composition according to claim 4, wherein the anti-PD-Ll antibody is CX-072, WBP-3155, KN035, A167, Cosibelimab, Atezolizumab, Avelumab, Durvalumab, Adebrelimab, or BMS-936559.

7. The pharmaceutical composition according to claim 1, wherein the fusion protein and the PD-1 or PD-L1 antagonist are comprised in a composite formulation.

8. The pharmaceutical composition according to claim 1, wherein the fusion protein and the PD-1 or PD-L1 antagonist are administered simultaneously or sequentially.
